(19) Europäisches Patentamt
European Patent Office
Office européen des brevets

(11) Veröffentlichungsnummer: **0 409 055 A1**

## (12) EUROPÄISCHE PATENTANMELDUNG

(21) Anmeldenummer: **90113195.3**

(22) Anmeldetag: **11.07.90**

(51) Int. Cl.5: **C07D 501/36**, C07D 277/36, C07D 277/56, A61K 31/545

(30) Priorität: **15.07.89 DE 3923541**

(43) Veröffentlichungstag der Anmeldung: **23.01.91 Patentblatt 91/04**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Adam, Friedhelm, Dr.**
**Rheingaustrasse 46**
**D-6238 Hofheim am Taunus(DE)**
Erfinder: **Dürckheimer, Walter, Dr.**
**Im Lerchenfld 45**
**D-6234 Hattersheim am Main(DE)**
Erfinder: **Scheunemann, Karl-Heinz, Dr.**
**Geisenheimer Strasse 88**
**D-6000 Frankfurt am Main 71(DE)**
Erfinder: **Isert, Dieter, Dr.**
**Hamburger Strasse 1**
**D-6236 Eschborn(DE)**
Erfinder: **Seibert, Gerhard, Prof.Dr.**
**Gläserweg 21**
**D-6100 Darmstadt(DE)**

# (54) Cephalosporinderivate und Verfahren zu ihrer Herstellung.

(57) Cephalosporinderivate der allgemeinen Formel

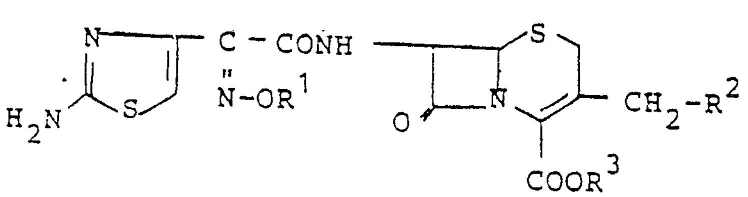

gegen bakterielle Infektionen wirksame pharmazeutische Präparate, die solche Cephalosporinderivate enthalten, Verfahren zur Herstellung der Cephalosporinderivate, Verwendung der Cephalosporinderivate zur Herstellung eines Arzneimittels zur Bekämpfung bakterieller Infektionen, sowie Ausgangsprodukte zur Herstellung der Cephalosporinderivate

EP 0 409 055 A1

## CEPHALOSPORINDERIVATE UND VERFAHREN ZU IHRER HERSTELLUNG

Die Erfindung betrifft neue Cephalosporinderivate und Verfahren zu ihrer Herstellung, insbesondere Cephemderivate, die in 3'-Stellung des Cephemrings durch eine bislang in Verbindung mit Cephalosporinen noch nicht beschriebenen 5-Thio-1,3-thiazolring substituiert sind und die eine sehr gute antimikrobielle Wirkung gegen Gram-positive und Gram-negative Bakterien besitzen und deshalb als Arzneimittel zur Behandlung von mikrobiellen Infektionen gut geeignet sind. Die Erfindung betrifft weiterhin ein Verfahren zu ihrer Herstellung, sie enthaltende pharmazeutische Zubereitungen und deren Herstellung und Verwendung zur Bekämpfung bakterieller Infektionen.

Gegenstand der Erfindung sind daher Cephalosporinderivate der allgemeinen Formel I

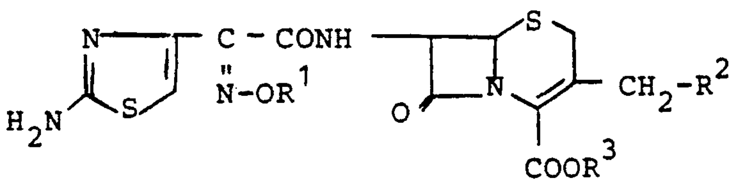

(I)

und deren physiologisch verträgliche Salze und Säureadditionssalze, worin

$R^1$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_4$-$C_7$-Cycloalkenyl, die Gruppe

$$(CH_2)_n(\overset{R^3}{\underset{R^4}{C}})_mR^5 ,$$

worin m oder n jeweils 0 oder 1, $R^3$ und $R^4$ gleich oder verschieden sein können und Wasserstoff, Aryl, eine $C_1$-$C_4$-Alkylgruppe, oder zusammen mit dem Kohlenstoffatom, an das sie gebunden sind, eine Methylen- oder eine $C_3$-$C_7$-Cycloalkylidengruppe bilden;

$R^5$ eine Gruppe -$CO_2R^6$, in der $R^6$ Wasserstoff, $C_1$-$C_4$-Alkyl oder ein Äquivalent eines Alkalimetalls, Erdalkalimetalls, Ammonium oder einer organischen Aminbase bedeutet;

$R^2$ filr einen 5-Thio-1,3-thiazolrest der Formel II

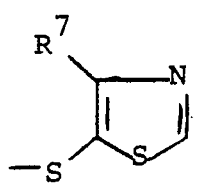

(II)

steht, worin $R^7$ die Bedeutung besitzt von Wasserstoff, Carboxy, $C_1$-$C_4$-Alkoxycarbonyl, Carbamoyl, in den die Aminogruppe gegebenenfalls auch einfach oder zweifach durch $C_1$-$C_4$-Alkyl substituiert sein kann oder auch zwei der am Stickstoff stehenden Alkylgruppen zu einem Di- bis Pentamethylenring geschlossen sein können und

$R^3$ Wasserstoff, ein physiologisch verträgliches Kation oder eine leicht abspaltbare Estergruppe bedeutet und in denen die $R^1$O-Gruppe in syn-position steht.

Die vorliegende Erfindung ist bevorzugt auf Verbindungen gerichtet, in denen $R^1$, $R^7$ und $R^3$ die folgenden Bedeutungen besitzen:

$R^1$ = Wasserstoff

$C_1$-$C_4$-Alkyl, wie z.B. Methyl, Ethyl, Propyl, Isopropyl, Butyl, insbesondere Methyl und Ethyl, vorzugsweise Methyl,

$C_1$-$C_4$-Alkyl, das ein- oder mehrfach, insbesondere 1- bis 3-fach substituiert ist

durch Halogen, wie z.B. Chlor oder Fluor, insbesondere Fluor
durch $C_1$-$C_6$-, vorzugsweise $C_1$-$C_4$-Alkylthio, wie z.B. Methylthio, Ethylthio, Propylthio, Butylthio, insbesondere Methylthio und Ethylthio,
durch $C_1$-$C_6$-, vorzugsweise $C_1$-$C_4$-Alkyloxy, wie z.B. Methoxy, Ethoxy, Propoxy, Isopropoxy und Butoxy, insbesondere Methoxy und Ethoxy,
durch Aryl, insbesondere Phenyl oder
durch Heteroaryl, wie z.B. Pyridyl, Furyl, $C_2C_6$-, vorzugsweise $C_2$-$C_4$-Alkenyl, wie z.B. Vinyl oder Allyl, insbesondere Allyl, das auch ein- oder mehrfach, insbesondere einfach substituiert sein kann durch Halogen, wie z.B. Chlor und Brom, insbesondere durch Chlor,
$C_2$-$C_3$-Alkinyl, wie z.B. Ethinyl oder Propargyl, insbesondere Propargyl,
$C_3$-$C_7$-, vorzugsweise $C_3$-$C_5$-Cycloalkyl, wie z.B. Cyclopropyl, Cyclobutyl und Cylopentyl, insbesondere Cyclobutyl und Cyclopentyl,
$C_4$-$C_7$-, vorzugsweise $C_5$-$C_6$-Cycloalkenyl, wie z.B. Cyclopentyl und Cyclohexyl, insbesondere Cyclopentenyl
oder die Gruppe

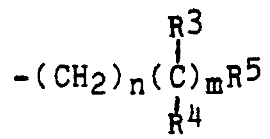

worin
m und n jeweils 0 oder 1 bedeuten und wobei vorzugsweise entweder m oder n für 0 steht,
$R^3$ und $R^4$, die gleich oder verschieden sein können, die Bedeutung besitzen von
Wasserstoff,
Aryl, insbesondere Phenyl,
$C_1$-$C_4$-Alkyl, wie z.B. Methyl, Ethyl, Propyl, Butyl, insbesondere Methyl,
oder worin
$R^3$ und $R^4$ zusammen mit dem Kohlenstoffatom an das sie gebunden sind, eine Methylen- oder eine $C_3$-$C_7$-, vorzugsweise $C_3$-$C_5$-Cycloalkylidengruppe bilden, wie z.B. Cyclopropyliden, Cyclobutyliden und Cyclopentyliden, insbesondere Cyclopropyliden,
$R^5$ für eine Gruppe -$COOR^6$ steht, in der $R^6$ Wasserstoff, $C_1$-$C_4$-Alkyl, wie z.B. Methyl, Ethyl, Propyl, Isopropyl, Butyl, Isobutyl und tert.-Butyl, insbesondere tert.-Butyl sein kann oder ein Äquivalent eines Alkalimetalls, vorzugsweise Natrium und Kalium, eines Erdalkalimetalls, wie z.B. Calcium und Magnesium, insbesondere Calcium, von Ammonium oder einer oranischen Aminbase, wie z.B. Diisopropylamin, Dicyclohexylamin und Triethylamin, insbesondere Triethylamin.
Ganz besonders bevorzugt für $R^1$ sind Wasserstoff, $C_1$-$C_4$-Alkyl, insbesondere Methyl und CArboxymethyl.
$R^7$ hat bevorzugt die Bedeutung von
Wasserstoff,
Carboxy,
$C_1$-$C_4$-Alkoxycarbonyl, wie beispielsweise Methoxy-, Ethoxy-, Propoxy-, Isopropoxy-, Butoxy-, sec.-Butoxy- oder tert.-Butoxycarbonyl, insbesondere Methoxy-, Ethoxy- oder tert.-Butoxycarbonyl,
Carbamoyl,
Carbamoyl, das am Stickstoff ein- oder zweifach substituiert ist durch $C_1$-$C_4$-Alkyl, wie z.B. Methyl, Ethyl, Propyl, Isopropyl oder Butyl, vorzugsweise Methyl oder in dem zwei der am Stickstoff stehenden Alkylgruppen auch zu einem Di- bis Pentamethylenring, vorzugsweise einem Tetra- oder Pentamethylenring, insbesondere zu einem Tetramethylenring, geschlossen sein können.
Ganz besonders bevorzugt für $R^7$ sind Wasserstoff, Carboxy, $C_1$-$C_4$-Alkoxycarbonyl, insbesondere Methoxycarbonyl, Carbamoyl und Pyrrolidinocarbonyl.
$R^3$ steht bevorzugt für Wasserstoff, ein physiologisch verträgliches Kation oder eine der leicht abspaltbaren Estergruppen, die weiter unten genau beschrieben wird. Eine besonders bevorzugte Gruppe sind die niederen Alkanoyloxy-alkylester, wie z.B. der 2,2-Dimethylpropionyloxymethylester. Als physiologisch verträgliche Kationen kommen in Betracht Alkalikationen, vorzugsweise Natrium oder Kalium; Erdalkalikationen, vorzugsweise Magnesium und Calcium; Ammonium und organische Aminbasen, wie z.B. Diisopropylamin, Dicyclohexylamin und Triethylamin, insbesondere Triethylamin.

Gegenstand der Erfindung ist weiterhin ein Verfahren zur Herstellung von Verbindungen der allgemeinen Formel I und ihrer physiologisch verträglichen Salze und Säureadditionssalze, das dadurch gekennzeichnet ist, daß man

a) eine Verbindung der allgemeinen Formel III

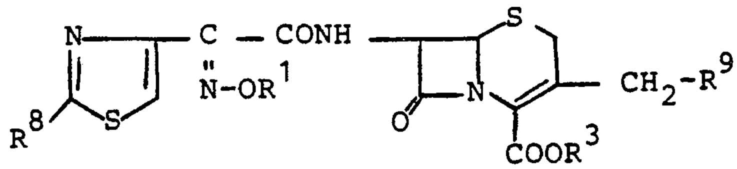

(III)

oder deren Salze, worin $R^1$ und $R^3$ die obengenannte Bedeutung hat, $R^8$ eine Amino- oder eine geschützte Aminogruppe bedeutet und $R^9$ eine durch 5-Thio-1,3-thiazol oder in Position 4 substituiertes 5-Thio-1,3-thiazol, die den Resten $R^2$ in der Formel (I) entsprechen, austauschbare Gruppe bedeutet, mit einem solchen 5-Mercapto-1,3-thiazol oder einem seiner 4-substituierten Derivate umsetzt, eine gegebenenfalls vorhandene Schutzgruppe abspaltet und falls erforderlich, das erhaltene Produkt in ein physiologisch verträgliches Salz oder Säureadditionssalz überführt, oder

b) eine 7-Amino-cephemverbindung der allgemeinen Formel IV

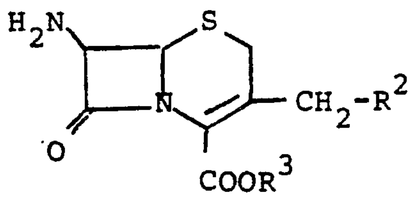

(IV)

oder deren Salze oder Säureadditionssalze, worin $R^2$ und $R^3$ die obgenannte Bedeutung haben und worin die Aminogruppe auch in Form eines reaktionsfähigen Derivats vorliegen kann, mit einer Thiazol-4-yl-2-syn oximessigsäure der allgemeinen Formel V,

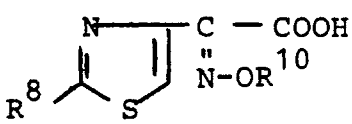

(V)

worin $R^8$ die obige Bedeutung besitzt und $R^{10}$ die Bedeutung von $R^1$ hat, wobei für den Fall, daß $R^1$ in Formel (I) Wasserstoff ist, $R^{10}$ eine Schutzgruppe bedeutet, oder mit einem aktivierten Derivat dieser Verbindung umsetzt, eine gegebenenfalls vorhandene Schutzgruppe abspaltet und falls erforderlich, das erhaltene Produkt in ein physiologisch verträgliches Salz oder Säureadditionssalz überführt.

Steht $R^3$ für eine leicht abspaltbare Estergruppe, so kann $R^3$ die Bedeutung von in der Penicillin- und Cephalosporinchemie üblichen Resten haben, wie z.B. niedere Alkylester, wie z.B. Methyl-, Ethyl-, Propyl-, Isopropyl-, Butyl-, Isobutyl-, tert.-Butyl-, Pentyl-, tert.-Pentyl-, Hexylester, niedere Alkenylester, wie z.B. Vinyl-, Allylester; niedere Alkinylester, wie z.B. Propargylester; niedere Alkoxyalkylester, wie z.B. Methoxymethyl-, Ethoxymethyl-, Isopropoxymethyl-, 1-Methoxyethyl-, 1-Ethoxyethylester; niedere Alkylthioalkylester, wie z.B. Methylthiomethyl-, Ethylthiomethyl-, Ethylthioethyl-, Isopropylthiomethylester; durch Amino und Carboxy substituierte niedere Alkylester, wie z.B. 2-Amino-2-carboxyethylester, 3-Amino-2-carboxypropylester; durch geschütztes Amino und geschütztes Carboxy substituierte niedere Alkylester, wie auch durch niederes Alkoxycarbonylamino und Mono-, Di- oder Triphenyl-alkoxycarbonyl substituierte niedere Alkylester, wie z.B. 2-tert.-Butoxycarbonylamino-2-benzhydryloxycarbonylethyl-, 3-tert.-Butoxycarbonylamino-3-benzhydryloxycarbonylpropylester; niedere Mono-, Di- oder Trihalogenalkylester, wie z.B. 2-Jodethyl-, 2,2,2-Trichlorethylester; niedere Alkanoyloxyalkylester, wie z.B. Acetoxymethyl-, Propionyloxymethyl-, Butyryloxymethyl-, Isobutyryloxymethyl-, Valeryloxymethyl-, Pivaloyloxymethyl-, Hexanoyloxymethyl-, 2-Acetoxymethyl-, 2-Propionyloxyethyl-, Butyryloxyethyl-, Isobutyryloxyethyl-, Valeryloxyethyl-, Pivaloyloxyethyl-, Hexanoyloxyethylester; niedere Alkoxycarbonyloxyalkylester, wie z.B. Methoxycarbonyloxyethyl-, Ethoxycarbonyloxyethyl-, Propoxycarbonyloxyethyl-, Isopropoxycarbonyloxyethylester; Cycloalkoxycarbonyloxyethylester, wie z.B. CYclopentyloxycarbonyloxyethyl-, Cyclohexyloxycarbonyloxyethylester; niedere Alkylsulfonylester, wie z.B. Mesylmethyl-, 2-Mesylethylester; niedere Mono-, Di- oder Triphenylalkylester, die eine oder mehrere Substituenten tragen können, wie z.B. Benzyl-, 4-Nitrobenzyl-, Phenethyl-, Benzhydryl-, Trityl-, bis-(Methoxyphenyl)-methyl-, 3,4-Dimethoxybenzyl-, 4-Hydroxy-3,5-di-tert.-butylbenzylester; Arylester, die einen oder mehrere Substituenten tragen können, wie z.B. Phenyl-, Tolyl-, tert.-Butylphenyl-, Xylyl-, Mesityl-, Cumenyl-, Salicylester; heterocyclische Ester, wie z.B. Phthalidylester.

Der bei der Definition von $R^3$ auftretende Begriff "niedere" bedeutet für gesättigte Reste $C_1$-$C_6$-, vorzugsweise $C_1$-$C_4$-Kohlenstoffatome, für ungesättigte Reste $C_2$-$C_6$-, vorzugsweise $C_2$-$C_4$-Kohlenstoffatome.

Soll die Herstellung der Verbidungen der allgemeinen Formel (I) durch nucleophilen Austausch von $R^9$ in den Verbindungen der allgemeinen Formel (III) durch 5-Mercapto-1,3-thiazol oder eines seiner angegebenen Derivate erfolgen, so kommen als Reste $R^9$ insbesondere in Betracht Acyloxyreste von niederen aliphatischen Carbonsäuren, vorzugsweise mit 1 bis 4 C-Atomen, wie z.B. Acetoxy oder Propionyloxy, insbesondere Acetoxy, die gegebenenfalls substituiert sein können, wie z.B. Chloracetoxy oder Acetylacetoxy. Für $R^9$ kommen auch andere Gruppen in Betracht, wie beispielsweise Halogen insbesondere Chlor, Brom oder Jod, oder Carbamoyloxy.

Bevorzugt werden bei der nucleophilen Austauschreaktion Ausgangsverbindungen der allgemeinen Formel (III), in denen $R^9$ für Acetoxy steht, eingesetzt, oder deren Salze, wie z.B. das Natrium- oder Kaliumsalz. Bei Verwendung von Verbindungen der allgemeinen Formel (IV) können diese ebenfalls durch nucleophilen Austausch in an sich bekannter Weise, beispielsweise aus der 7-Aminocephalosporansäure oder aus der an der Aminogruppe geschützten 7-Aminocephalosporansäure erhalten werden. Die nucleophile Austauschreaktion wird in einem Lösungsmittel, vorzugsweise in Wasser oder in einem Gemisch aus Wasser und einem mit Wasser leicht mischbaren organischen Lösungsmittel, wie z.B. Aceton, Dioxan, Acetonitril, Dimethylformamid, Dimethylsulfoxid oder Ethanol durchgeführt. Die Reaktionstemperatur liegt im allgemeinen im Bereich von etwa 10 bis 100 °C, vorzugsweise zwischen 20 und 80 °C. Die Basenkomponente wird in Mengen zugegeben, die zwischen etwa äquimolaren Mengen und einem bis zu etwa 15-fachen Überschuß liegt. Die Reaktion wird vorteilhaft in der Nähe des Neutralpunktes, vorzugsweise bei einem pH-Wert im Bereich von etwa 5 bis etwa 8 durchgeführt, z.B. in Form des Natriumsalzes von 5-Mercapto-1,3-thiazol oder eines seiner Derivate.

Liegt die Gruppe $R^8$ als geschützte Aminofunktion vor, so eignen sich Aminoschutzgruppen die in der Peptidchemie bekannten Schutzgruppen, beispielsweise tert.-Butyl, tert.-Amyl, Benzyl, p-methoxybenzyl, Trityl, Benzhydryl, vorzugsweise Trityl; wie beispielsweise Trimethylsilyl; gegebenenfalls substituiertes aliphatisches Acyl, wie z.B. Formyl, Chloracetyl, Bromacetyl, Trichloracetyl und Trifluoracetyl, vorzugsweise Formyl; oder gegebenenfalls substituiertes Alkoxycarbonyl wie beispielsweise Trichlorethoxycarbonyl, Benzyloxycarbonyl oder tert.-Butyloxycarbonyl, vorzugsweise tert.-Butyloxycarbonyl und Benzyloxycarbonyl oder Dimethylaminomethylen.

Hat $R^1$ in der allgemeinen Formel I die Bedeutung von Wasserstoff, so steht $R^{10}$ in der allgemeinen Formel (V) für eine aus der Peptid- und Cephalosporinchemie bekannte leicht abspaltbare Gruppe, vorzugsweise für Benzhydryl, Trityl, Tetrahydropyranyl oder 1-methoxy-1-methyl-ethyl. Besonders bevorzugt für $R^{10}$ ist Trityl und 1-Methoxy-1-methyl-ethyl.

Die Schutzgruppen können nach der Austauschreaktion in an sich bekannter Weise abgespalten werden, z.B. die Trityl-und 1-Methoxy-1-methyl-ethyl-Gruppe mittels einer Säure, wie z.B. Essigsäure, Trifluoressigsäure, Ameisensäure oder die Benzyloxycarbonylgruppe hydrogenolytisch.

Die Reaktionsprodukte der Formel (I) können aus der Reaktionsmischung in üblicher Weise, z.B. durch Gefriertrocknen der Wasserphase, Chromatographie oder auch durch Ausfällen bei pH 3 - 4 isoliert werden.

Die Acylierung der Verbindungen der allgemeinen Formel (IV) oder von deren Additionssalzen, beispielsweise mit Chlorwasserstoffsäure, Bromwasserstoffsäure oder Maleinsäure, wird mit Carbonsäure der allgemeinen Formel (V) oder mit einem reaktionsfähigen Derivat einer solchen Säure durchgeführt. In manchen Fällen ist es dabei von Vorteil, die 2-Aminogruppe und die Oximgruppe in den Verbindungen der allgemeinen Formel (V) vor der Umsetzung zu schützen. Als Aminoschutzgruppen eignen sich die vorstehend für $R^8$ und $R^{10}$ beschriebenen Schutzgruppen. Die Schutzgruppe kann nach der Acylierung in an sich bekannter Weise abgespalten werden, z.B. die Tritylgruppe und 1-Methoxy-1-methyl-ethyl-Gruppe mittels einer Carbonsäure, wie z.B. Ameisensäure oder Trifluoressigsäure, oder die Chloracetylgruppe mittels Thioharnstoff. Werden die Carbonsäuren der allgemeinen Formel V sowie ihre an der Amino- und Oximgruppe geschützten Derivate selbst als Acylierungsmittel eingesetzt, so wird zweckmäßig in Gegenwart eines Kondensationsmittel, beispielsweise eines Carbodiimids, wie beispielsweise N,N′-Dicyclohexylcarbodiimid, gearbeitet.

Die Aktivierung der Carbonsäuren der allgemeinen Formel (V) kann in besonders günstiger Weise durch Behandlung mit bestimmten Carbonsäureamiden und beispielsweise Phosgen, Phosphorpentachlorid, Tosylchlorid, Thionylchlorid oder Oxalylchlorid erfolgen, wie sie in der Deutschen Patentschrift 28 04 040 beschrieben wird.

Als aktivierte Derivate der Carbonsäuren der allgemeinen Formel (V) eignen sich insbesondere auch Halogenide, vorzugsweise Chloride, die in an sich bekannter Weise durch Behandlung mit Halogenierungsmittel, wie z.B. Phosphorpentachlorid, Phosgen oder Thionylchlorid unter für die Cephalosporinchemie literaturbekannten, schonenden Reaktionsbedingungen erhalten werden.

Als aktivierte Derivate der Carbonsäuren der allgemeinen Formel (V) eignen sich ferner die Anhydride und gemischte Anhydride, Azide und aktivierten Ester, vorzugsweise mit p-Nitrophenol, 2,4-Dinitrophenol, Methylcyanhydrin, N-Hydroxsuccinimid und N-Hydroxyphthalimid, insbesondere diejenigen mit 1-Hydroxybenzotriazol und 6-Chlor-1-hydroxybenzotriazol. Als gemischte Anhydride sind besonders geeignet solche mit niederen Alkansäuren, wie z.B. Essigsäure, und besonders bevorzugt solche mit substituierten Essigsäuren, wie z.B. Trichloressigsäure, Pivalinsäure oder Cyanessigsäure. Besonders geeignet sind aber auch die gemischten Anhydride von Sulfonsäuren, wie beispielsweise Benzosulfonsäure, Toluolsulfonsäure oder Ethylbenzolsulfonsäure oder Kohlensäurehalbester, die man beispielsweise durch Umsetzung der Carbonsäuren der Formel (V), in denen die Aminogruppe geschützt ist, mit Chlorameisensäure-benzylester, -p-nitrobenzylester, -isobutyl-ester, -ethylester oder -allylester gewinnt. Die aktivierten Derivate können als isolierte Substanzen, aber auch in situ umgesetzt werden.

Im allgemeinen erfolgt die Umsetzung der Cephemderivate der allgemeinen Formel (IV) mit einer Carbonsäure der allgemeinen Formel (V) oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Ether, wie z.B. Diethylether, vorzugsweise Tetrahydrofuran und Dioxan; Ketone, wie vorzugsweise Aceton und Butanon; Amide, wie vorzugsweise Dimethylformamid und Dimethylacetamid, oder Pyridin. Es kann sich aber auch als vorteilhaft erweisen, Gemische der genannten Lösungsmittel zu verwenden. Dies ist oftmals dann der Fall, wenn die Cephemverbindung der allgemeinen Formel (IV) mit einem in situ erzeugten aktivierten Derivat einer Carbonsäure der Formel (V) umgesetzt wird.

Die Umsetzung von Cephemverbindungen der Formel (IV) mit Carbonsäuren der Formel (V) bzw. deren aktivierten Derivaten kann in einem Temperaturbereich von etwa -80 bis etwa +80 °C, vorzugsweise zwischen -30 und +50 °C, insbesondere zwischen -20 °C und Raumtemperatur erfolgen.

Die Reaktionsdauer hängt von den Reaktanten, der Temperatur und dem Lösungsmittel bzw. dem Lösungsmittelgemisch ab und liegt normalerweise zwischen etwa 1/4 und etwa 72 Stunden.

Die Reaktion mit Säurehalogeniden kann gegebenenfalls in Gegenwart eines säurebindenden Mittels zur Bindung des freigesetzten Halogenwasserstoffs durchgeführt werden. Als solche eignen sich insbesondere tertiäre Amine, wie z.B. Triethylamin oder Dimethylanilin; anorganische Basen, wie z.B. Kaliumcarbonat oder Natriumcarbonat; Alkylenoxide, wie z.B. Propylenoxid. Auch die Anwesenheit eines Katalysators, wie z.B. von Dimethylaminopyridin, kann gegebenenfalls von Vorteil sein.

Liegt in den Verbindungen der allgemeinen Formel (IV) die Aminogruppe in Form eines reaktionsfähigen Derivats vor, so kann es sich um ein solches handeln, wie es aus der Literatur für Amidierungen bekannt ist. So kommen beispielsweise Silylderivate in Betracht, die bei der Umsetzung von Verbindungen der allgemeinen Formel (IV) mit einer Silylverbindung gebildet werden, wie z.B. Trimethylchlorsilan oder Bis-(trimethylsilyl)-acetamid. Wird die Umsetzung mit einer solchen, an der Aminogruppe aktivierten Verbindung durchgeführt, so ist es zweckmäßig, die Reaktion in einem inerten Lösungsmittel, wie z.B. Methylenchlorid, Tetrahydrofuran oder Dimethylformamid durchzuführen.

Als physiologisch verträgliches Säureadditionssalz der Verbindungen der allgemeinen Formel (I) seien beispielsweise erwähnt solche mit Chlorwasserstoffsäure, Bromwasserstoffsäure, Salpetersäure, Phosphorsäure, Schwefelsäure oder organischen Säuren, wie z.B. Methanolsulfonsäure, p-Toluolsulfonsäure, p-Ethylbenzolsulfonsäure oder Maleinsäure.

Die phsiologisch verträglichen Salze und Säureadditionssalze können in an sich bekannter Weise erhalten werden durch Zusammengeben der Verbindungen der allgemeinen Formel (I) und der Säure- bzw. Basen-Komponente in einem geeigneten Lösungsmittel, beispielsweise in wäßrig-alkoholischer Lösung.

Gegenstand der Erfindung sind auch die Zwischenprodukte der allgemeinen Formel (VI)

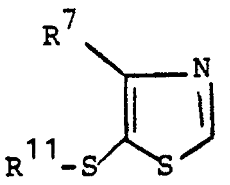

(VI)

worin $R^7$ die in Formel II genannte Bedeutung besitzt und $R^{11}$ filr Wasserstoff, Natrium oder Kalium steht.

5-Thio-thiazole der allgemeinen Formel (VI)

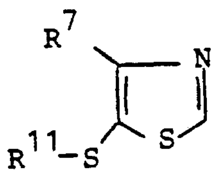

(VI)

in der $R^7$ die obige Bedeutung hat, jedoch nicht für Wasserstoff stehen kann, und $R^{11}$ Wasserstoff, Natrium oder Kalium sein kann, können dadurch erhalten werden, daß man Verbindungen der allgemeinen Formel (VII)

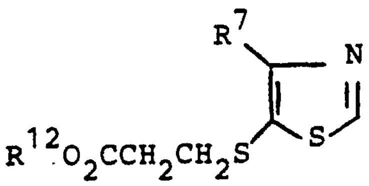

(VII)

in der $R^7$ die obige Bedeutung - mit Ausnahme von Wasserstoff - hat und $R^{12}$ Wasserstoff oder ein $C_1$-$C_4$-Alkylrest, wie beispielsweise Methyl, Ethyl, Propyl, Isopropyl oder Butyl, vorzugsweise Methyl und Ethyl, besonders aber Ethyl sein kann, mit Basen bei erhöhter Temperatur umsetzt und das entsprechende Mercaptid beispielsweise durch Fällen mit einem organischen Lösungsmittel oder nach Ansäuern die freie Mercaptoverbindung ($R^{11}$ = H) isoliert.

Die Produkte der Formel (VI) können nach Isolierung oder auch direkt ohne weitere Reinigung mit 7-ACS in an sich bekannter Weise zu den Verbindungen der allgemeinen Formel (III) umgesetzt werden.

Die Reaktion wird bevorzugt in einem organischen Lösungsmittel, vorzugsweise in Methanol, Ethanol oder einem Gemisch aus Wasser und einem der Alkohole durchgeführt. Bei der Verwendung von Alkoholen als Lösungsmittel wird zweckmäßigerweise als Base eine Lösung des entsprechnden Natrium- oder Kaliumalkoholats in demselben Lösungsmittel verwendet. Die Reaktionstemperatur liegt im allgemeinen zwischen 30 und 100 °C, vorzugsweise zwischen 60 und 80 °C. Die Basenkomponente wird in Mengen zugegeben, die zwischen etwa äquimolaren Mengen und einem bis zu dreifachen Überschuß liegt. Das Mercaptid kann beispielsweise auch durch Zugabe von Natronlauge in wäßrig-alkoholischer Lösung erhalten werden.

Die Herstellung von Verbindungen der allgemeinen Formel (VI), in der $R^7$ gleich Wasserstoff ist, erfolgt aus Verbindungen der allgemeinen Formel (VIII)

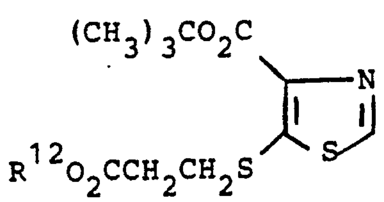

(VIII)

in der $R^{12}$ die obige Bedeutung hat, durch Behandlung mit organischen Säuren, wie beispielsweise

Trifluoressigsäure, Ameisensäure und Decarboxylierung der entstehenden Säure der allgemeinen Formel (IX)

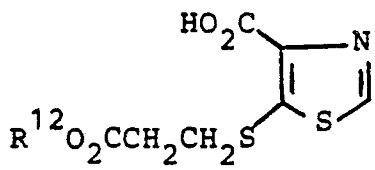

(IX)

in üblicher Weise, beispielsweise durch Erhitzen, gefolgt von einer Umsetzung mit Basen, nach den oben beschriebenen Bedingungen zum gewünschten Heterocyclus der allgemeinen Formel (X)

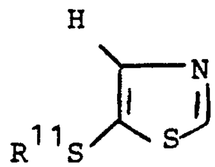

(X)

in der $R^{11}$ die obige Bedeutung hat.

Die Herstellung der Verbindungen der allgemeinen Formel (VII) kann in Analogie zu einem literaturbekannten Verfahren, das in Liebigs Annalen, S. 2122-2125 (1986), beschrieben ist, erfolgen. Beispiele von Verbindungen der allgemeinen Formel (VII) sind in der EP 0 194 572 beschrieben.

Die erfindungsgemäß erhaltenen Verbindungen der allgemeinen Formel (I) und ihre physiologisch verträglichen Salze und Säureadditionssalze zeigen bemerkenswert gute antibakterielle Wirksamkeit sowohl gegen Gram-positive als auch Gram-negative bakterielle Keime.

Auch gegen penicillinase- und cephalosporinasebildende Bakterien sind die Verbindungen der Formel I unerwartet gut wirksam. Da sie zudem günstige toxikologische und pharmakologische Eigenschaften zeigen, stellen sie wertvolle Chemotherapeutika dar.

Die Erfindung betrifft somit auch Arzneipräparate zur Behandlung von mikrobiellen Infektionen, die durch einen Gehalt an einer oder mehreren der erfindungsgemäßen Verbindungen charakterisiert sind.

Die Verbindungen der allgemeinen Formel (I) und ihre physiologisch verträglichen Salze und Säureadditionssalze können oral, intramuskulär oder intravenös verabfolgt werden. Arzneipräparate, die eine oder mehrere Verbindungen der allgemeinen Formel I als Wirkstoff enthalten, können hergestellt werden, indem man die Verbindungen der Formel (I) mit einem oder mehreren pharmakologisch verträglichen Trägerstoffen oder Verdünnungsmittel, wie z.B. Füllstoffen, Emulgatoren, Gleitstoffen, Geschmackskorrigentien, Farbstoffen oder Puffersubstanzen vermischt und in eine geeignete galenische Zubereitungsform bringt, wie beispielsweise Tabletten, Dragees, Kapseln, oder eine zur parenteralen Applikation geeignete Suspension oder Lösung.

Als Träger- oder Verdünnungsmittel seien beispielsweise erwähnt Traganth, Milchzucker, Talkum, Agar-Agar, Polyglykole, Ethanol und Wasser. Puffersubstanzen sind beispielsweise organische Verbindungen wie z.B. N,N'-Dibenzylethylendiamin, Diethanolamin, Ethylendiamin, N-Methylglucamin, N-Benzylphenethylamin, Diethylamin, Tris(hydroxymethyl)-aminoethan oder anorganische Verbindungen, wie z.B. Phosphatpuffer, Natriumbicarbonat oder Natriumcarbonat. Für die parenterale Applikation kommen vorzugsweise Suspensionen oder Lösungen in Wasser mit oder ohne Puffersubstanzen in Betracht. Es ist auch möglich, die Wirkstoffe als solche ohne Träger- oder Verdünnungsmittel in geeigneter Form, beispielsweise in Kapseln, zu applizieren.

Geeignete Dosen der Verbindungen der allgemeinen Formel I oder ihrer physiologisch verträglichen Salze oder Säureadditionssalze liegen bei etwa 0,4 bis 10 g/Tag, vorzugsweise bei 0,5 bis 4 g/Tag, für einen Erwachsenen von etwa 80 kg Körpergewicht.

Es können Einzel- oder im allgemeinen Mehrfachdosen verabreicht werden, wobei die Einzeldosis den Wirkstoff in einer Menge von etwa 50 bis 1000 mg, vorzugsweise von etwa 100 bis 500 mg, enthalten kann.

Die folgenden Ausführungsbeispiele für erfindungsgemäß herstellbare syn-Verbindungen dienen zur weiteren Erläuterung der Erfindung, schränken sie jedoch nicht darauf ein.

## Herstellung von Ausgangsubstanzen

### Herstellungsbeispiel 1

5-[2-(Methoxycarbonyl)ethylthio]thiazol-4- carbonsäuremethylester

Zu einer Suspension von 10 g (88 mmol) Kalium-tert.-butylat in 80 ml wasserfreiem Tetrahydrofuran tropfte man bei -40°C eine Lösung von 7.9 g (80 mmol) Isocyanessigsäuremethylester in 60 ml wasserfreiem Tetrahydrofuran. Anschließend Kéhlte man auf -60°C ab und tropfte eine Lösung von 4.8 ml (80 mmol) Schwefelkohlenstoff in 60 ml wasserfreiem Tetrahydrofuran zu, wobei die Temperatur unter -50°C gehalten wurde. Nach beendeter Zugabe ließ man den Ansatz langsam auf 10°C kommen und gab 13.4 g (80 mmol) 3-Brom-propionsäure-methylester zu und rührte 2 h bei Raumtemperatur nach. Nach Abzug des Lösungsmittels wurde das Rohprodukt in Methylenchlorid aufgenommen, mit Wasser gewaschen, getrocknet ($MgSO_4$) und eingeengt. Nach Umkristallisation aus Essigester/Cyclohexan (1 + 1) wurden 13.8 g (66 %) der Titelverbindung erhalten.

Fp.: 74 - 75°C

$^1$H-NMR (60 MHz, DMSO-$d_6$): $\delta$(ppm) = 2.8-3.3 (4H,m,2x$CH_2$), 3.6 und 3.8 (6H,s,2x$OCH_3$), 9.1 (1H,s,CH).

Analog zu Herstellungsbeispiel 1 wurden die folgenden Verbindungen hergestellt:

### Beispiel 2

5-[(2-(Methoxycarbonyl)ethylthio]thiazol-4-carbonsäureamid:

Ausbeute: 46 %

Fp.: 151 - 152°C (Dioxan)

$^1$H-NMR (60 MHz, DMSO): $\delta$(ppm)= 2.5-3.15 (4H,m,2x$CH_2$), 3.66 (3H,s,$OCH_3$) 7.2-7.6 (2H,bs,$NH_2$), 8.6 (1H,s,CH)

### Beispiel 3

5-[(2-(Methoxycarbonyl)ethylthio]-thiazol-4-carbonsäure-tert-butylester

Ausbeute: 66 %

Fp.: 62 - 63°C ($Et_2O$)

$^1$H-NMR (60 MHz, DMSO): $\delta$(ppm) = 1.6 (9H,s,tert.Butyl), 2.5-3.2 (4H,m,2x$CH_2$), 3.66 (3H,s,$OCH_3$), 8.6 (1H,s,CH).

### Beispiel 4

5-[(2-(Methoxycarbonyl)ethylthio]-thiazol-4-carbonsäurepyrrolidinid

Ausbeute: 61 %

Fp.: 78 - 80°C

$^1$H-NMR (60 MHz, DMSO): $\delta$(ppm) = 1.7-2.1 (8H,m,Pyrrolidinyl-$CH_2$), 2.6-3.3 (4H,m,2x$CH_2$), 3.65 (3H,s,$OCH_3$), 8.6 (1H,s,CH).

### Herstellungsbeispiel 5

5-Mercaptothiazol-4-carbonsäuremethylester

10.6 g (40 mmol) der Titelverbindung aus Herstellungsbeispiel 1 wurden in 150 ml Methanol gelöst, 1.6 g (40 mmol) Natriumhydroxid zugegeben und 1 h unter Rückfluß erhitzt. Nach Einengen wurde der Rückstand in Essigester aufgenommen und mit 2N Salzsaure auf pH 2 eingestellt. Die organische Phase wurde getrocknet und eingeengt. Das so anfallende Rohprodukt wurde aus Essigester/Cyclohexan umkristallisiert. Man erhielt 5 g (71 %) der Titelverbindung.

Fp.: 100°C

$^1$H-NMR (60 MHz, DMSO-$d_6$): δ(ppm) = 3.65 (3H,s,$OCH_3$), 9.05 (1H,s,Thiazol-H).

**Herstellungsbeispiel 6**

5-Mercaptothiazol-4-carbonsäure

2.6 g (15 mmol) der Titelverbindung aus Herstellungsbeispiel 5 wurden in einem 1:1 Gemisch aus Wasser/Methanol gelöst, 1.2 g (30 mmol) Natriumhydroxid zugegeben und 3 h bei 80°C gehalten. Danach destillierte man Methanol ab, säuerte mit 2N Salzsäure an und extrahierte mit Methylenchlorid. Nach Trocknen ($MgSO_4$) und Abzug des Lösungsmittels wurde das anfallende Rohprodukt aus Aceton umkristallisiert. Man erhielt 1.4 g (58 %) der gewünschten Titelverbindung.

Fp.: 156 - 157°C

**Herstellungsbeispiel 7**

5-Mercaptothiazol-4-carbonsäureamid-Natriumsalz

8.9 g (36 mmol) der Titelverbindung aus Herstellungsbeispiel 2 wurden in 150 ml Methanol suspendiert und zu einer Lösung aus 0.83 g (36 mmol) Natrium in 75 ml Methanol gegeben und 1 h unter Rückfluß erhitzt. Danach wurde eingeengt, der Rückstand in wenig Methanol aufgenommen, die Lösung in 0.5 l Diisopropylether getropft, das Produkt abgesaugt und getrocknet ($CaCl_2$). Man erhielt 4.8 g (73 %) der gewünschten Titelverbindung, die ohne weitere Reinigung weiterverarbeitet wurde.

Herstellungsbeispiel 8

5-Mercaptothiazol-4-carbonsäurepyrrolidinid-Natriumsalz

15 g (50 mmol) der Titelverbindung aus Herstellungsbeispiel 4 wurden in einer Natriummethylatlösung, die aus 1.3 g (55 mmol) Natrium und 200 ml Methanol hergestellt wurde, gelöst und 40 Minuten unter Rückfluß erhitzt. Danach wurde eingeengt und das Rohprodukt ohne weitere Reinigung weiterverarbeitet.

**Herstellungsbeispiel 9**

5-Mercaptothiazol-Natriumsalz

**Stufe 1**

5-[2-(Methoxycarbonyl)ethylthio]thiazol-4-carbonsäure

7.8 g (26 mmol) der Titelverbindung aus Herstellungsbeispiel 3 wurden 30 Minuten in 100 ml

Trifluoressigsäure gerührt. Danach engte man ein, nahm den Rückstand in Methylenchlorid auf, rotierte erneut ein und versetzte den erhaltenen Kristallbrei mit Essig ester. Nach kurzem Rühren saugte man ab und wunsch mit einem Gemisch aus Ether/Petrolether (1:1). Man erhielt 6.2 g (97 %) der gewünschten Titelverbindung.

Fp.: 112 - 114°C

$^1$H-NMR (60 MHz, DMSO-$D_6$): δ(ppm) = 2.5-3.2 (4H,m,2x$CH_2$), 3.66 (3H, s,$OCH_3$), 9.0 (1H,s,CH).

**Stufe 2**

5-[2-(Methoxycarbonyl)ethylthio]thiazol

6.1 g (25 mmol) der Stufe 1 wurden ohne Lösungsmittel solange auf 180°C erhitzt bis die allmählich einsetzende Gasentwicklung beendet ist. Nach Abkühlen wurde der erstarrte Rückstand in wenig Essigester aufgenommen und chromatographiert ($SiO_2$; Aceton/Cyclohexan = 1+1). Nachfolgende Kugelrohrdestillation (Temperatur 180°C, 0.1 Torr) lieferte 2.8 g (56 %) der gewünschten Titelverbindung.

$^1$H-NMR (60 MHz, DMSO-$d_6$): δ(ppm) = 2.5-3.2 (4H,m,2x$CH_2$), 3.66 (3H, s,$OCH_3$), 7.9 und 9.25 (2H,s,Thiazol-H).

**Stufe 3**

5-Mercaptothiazol-Natriumsalz

9 g (45 mmol) 5-[2-(Methoxycarbonyl)ethylthio]-thiazol (Stufe 2) wurden in 90 ml Methanol gelöst und zu einer Lösung aus 1 g (45 mmol) Natrium in 180 ml Methanol getropft und anschließend 1.5 h unter Rückfluß gehalten. Nach Einengen wurde der Rückstand in wenig Methanol aufgenommen, die Lösung in 1 l Diethylether getropft, abgesaugt und getrocknet. Man erhielt 5.3 g (84 %) der gewünschten Titelverbindung, die ohne weitere Reinigung weiterverarbeitet wurde.

$^1$H-NMR (60 MHz, DMSO-$d_6$): δ(ppm) = 7.3 und 8.5 (2H,s,Thiazol-H).

**Herstellungsbeispiel 10**

7-Amino-3-[(4-methoxycarbonyl-5-thiazolyl)thiomethyl]ceph-3-em-4-carbonsäure

Zu einer Suspension von 2.7 g (10 mmol) 7-ACS in 20 ml Wasser wurden 1.7 g (2 eq.) Natriumhydrogencarbonat gegeben und nach Auflösung 1.75 g (10 mmol) 5-Mercaptothiazol-4-carbonsäuremethylester (Herstellungsbeispiel 5) zugesetzt. Die Lösung wurde danach 5 h bei 65°C gehalten und nach Abkühlen zweimal mit Essigester extrahiert. Die wässrige Phase wurde anschließend auf pH 3 angesäuert und das ausfallende Produkt abgesaugt und getrocknet. Man erhielt 2.6 g (68 %) der gewünschten Titelverbindung.

Fp.: 290°C (unter Zersetzung)

IR (KBr): 3420-2550, 1800, 1700, 1620, 1540, 1045 und 780 $cm^{-1}$

$^1$H-NMR (60 MHz, DMSO-$d_6$): δ(ppm) = 3.6 (2H,d,S-$CH_2$), 3.66 (3H,s,$OCH_3$), 4.2 (2H,d,-$CH_2$SHet), 4.6-5.1 (2H,m,H-6 und H-7).

Analog zu Herstellungsbeispiel 10 wurden die folgenden Verbindungen hergestellt:

**Herstellungsbeispiel 11**

7-Amino-3-[(4-carbamoyl-5-thiazolyl)thiomethyl]-ceph-3-em-4-carbonsäure

Fp.: 202°C (unter Zersetzung)

IR (KBr): 3500-2500, 1785, 1650, 1400, 1050 $cm^{-1}$.

$^1H$-NMR (60 MHz, DMSO-$d_6$): δ(ppm) = 3.6 (2H,d,S-$CH_2$), 4.15 (2H,d, -$CH_2$), 4.15 (2H,d,-$CH_2$S-Het), 4.6-5.1 (2H,m,H-6 und H-7), 7.5 (2H, breites s,$CONH_2$), 9.0 (1H,s,CH).

**Herstellungsbeispiel 12**

7-Amino-3-[(5-thiazol)-thiomethyl]ceph-3-em-4-carbonsäure

Fp.: 229°C (unter Zersetzung)
IR (KBr): 3500-2450, 1800, 1620, 1535, 1410, 1345, 800 $cm^{-1}$.
$^1H$-NMR (60 MHz, DMSO-$d_6$): δ(ppm) = 3.6 (2H,d,S-$CH_2$), 4.2 (2H,d, -$CH_2$S-Het), 4.6-5.1 (2H,m,H-6 und H-7), 7.95 (1H,s,H-4 Thiazol), 9.2 (1H,s,H-2 Thiazol)

**Herstellungsbeispiel 13**

7-Amino-3-[(4-pyrrolidinocarbonyl-5-thiazolyl)thiomethyl]ceph-3-em-4-carbonsäure

IR (KBr): 1805, 1630, 1345, 1045, 795 $cm^{-1}$.
$^1H$-NMR (60 MHz, DMSO-$d_6$): δ(ppm) = 1.66-2.0 (8H,m,Pyrrolidinocarbonyl), 3.6 (2H,d,S-$CH_2$), 4.1 (2H,d,$CH_2$S-Het), 4.6-5.0 (2H,m,H-6 und H-7), 9.2 (1H,s,Thiazol H).

**Herstellungsbeispiel 14**

7-Amino-3-[(4-hydroxycarbonyl-5-thiazolyl)thiomethyl]-ceph3-em-4-carbonsäure

IR (KBr): 3500-2500, 1790, 1700, 1390 $cm^{-1}$
Fp.: 220°C (unter Zersetzung)

**B) Ausführungsbeispiele**

**Beispiel 1**

7-[2-(2-Aminothiazol-4-yl)-2-(Z)-methoxyimino-acetamido]-3-[4-methoxycarbonyl-1,3-thiazol-5-yl)thiomethyl]-ceph-3-em-4-carbonsäure

Zu einer Lösung von 4.4 g (22 mmol) 2-Aminothiazol-4-yl-2-(Z)-methoxyiminoessigsäure in 200 ml DMF wurden nacheinander 3.7 g (24 mmol) N-Hydroxybenzotriazol (HOBT) und 4.95 g (24 mmol) Dicyclohexylcarbodiimid zugegeben und 4 h bei Raumtemperatur gerührt. Man saugte von entstandenem Dicyclohexylharnstoff ab und versetzte mit 7.75 g (20 mmol) 7-Amino-3-[(4-Methoxycarbonyl-1,3-thiazol-5-yl)thio methyl]ceph-3-em-4-carbonsäure (Herstellungsbeispiel 10) und rührte 10 h bei Raumtemperatur nach. Danach saugte man ab und zog das Lösungsmittel i. Vak. (0.1 Torr) ab. Der Rückstand wurde in 100 ml Wasser aufgenommen, mit Essigester überschichtet und durch Zugabe von Natriumhydrogencarbonat in Lösung gebracht. Die Phasen wurden sodann getrennt und die wässrige Lösung mit 2N Salzsäure auf pH 3 gestellt. Nach 15 Minuten Nachrühren wurde das Produkt abgesaugt und i. Vak. getrocknet (Phosphorpentoxid). Man erhielt 8.6 g (75 %) der gewünschten Titelverbindung.
Fp.: 170°C (unter Zersetzung)
IR (KBr): 1780, 1530, 1265, 1045 $cm^{-1}$
$^1H$-NMR (270 MHz, DMSO-$d_6$): δ(ppm) = 3.57 und 3.75 (2H, AB, J=18Hz,$SCH_2$), 3.83 und 3.85 (6H,2xs,$CO_2CH_3$ und N-$OCH_3$), 4.15 und 4.32 (2H,AB,J=12Hz,3'-$CH_2$), 5.15 (1H,d,J=6Hz,6-H), 5.78 (1H,dd,J=6Hz,7-H), 6.73 (1H,s,Thiazol-H),7.2 (2H,s,$NH_2$), 9.05 (1H,s,-S-CH=N), 9.62 (1H,d,NH).

**Ausführungsbeispiel 2**

7-[2-(2-Aminothiazol-4-yl)-2-(Z)-methoxyimino-acetamido]-3-[(4-carbamoyl-1,3-thiazol-5-yl)thiomethyl]ceph-3-em-4-carbonsaure

Die Herstellung erfolgte analog zu Ausführungsbeispiel 1.
Fp.:191°C (unter Zersetzung)
IR (KBr): 1775, 1660, 1045 $cm^{-1}$.
$^1$H-NMR (270 MHz,DMSO-$d_6$): δ(ppm) = 3.54 und 3.74 (2H,AB,J = 18Hz, $SCH_2$), 3.82 (3H,s,$OCH_3$), 4.17 und 4.3 (2H,AB,J = 12Hz,3′-$CH_2$), 5.15 (1H,d,J = 6.5 Hz,6-H), 5.77 (1H,dd,J = 6.5 Hz,7-H), 6.75 (1H,s,Thiazol-H), 7.2 (2H,s,$NH_2$ von Thiazol), 9.05 (1H,s,S-CH = N-), 9.62 (1H,d,NH).

Ausführungsbeispiel 3

7-[2-(2-Aminothiazol-4-yl) -2-(Z)-methoxyimino-acetamido]-3-[(1,3-thiazol-5-yl)thiomethyl]ceph-3-em-4-carbonsäure

Die Herstellung erfolgte analog zu Ausführungsbeispiel 1.
IR (KBr): 1780, 1660, 1045 $cm^{-1}$.
$^1$H-NMR (270MHz,DMSO-$d_6$): δ(ppm) = 3.5 und 3.72 (2H,AB,J = 18Hz,$SCH_2$), 3.83 (3H,s,$OCH_3$), 4.14 und 4.32 (2H,AB,J = 12Hz,3′-$CH_2$), 5.15 (1H,d,J = 6Hz,6-H), 5.77 (1H,dd,J = 6Hz,7-H), 6.75 (1H,x,Thiazol H), 7.2 (2H,s,$NH_2$), 8.05 (1H,s,2-H von Het.), 9.21 (1H,s,4-H von Het.), 9.6 (1H,d,NH).

**Ausführungsbeispiel 4**

7-[2-(2-Aminothiazol-4-yl)-2-(Z)-hydroxyimino-acetamido]-3-[(4-methoxycarbonyl-1,3-thiazol-5-yl)thiomethyl]-ceph-3-em-4-carbonsäure

**Stufe 1**

7-[2-(2-Tritylaminothiazol-4-yl)-2-(Z)-trityl-oximino-acetamido]-3-[4-methoxycarbonyl-1,3-thiazol-5-yl)-thiomethyl]ceph-3-em-4-carbonsäure

Zu einer Lösung von 1.0 g (1.5 mmol) 2-(2-Tritylaminothiazol-4-yl)-2-(Z)-trityl-oximino-essigsäure in 20 ml wasser freiem Methylenchlorid wurden 0.28 mg Triethylamin zugetropft, auf -20°C gekühlt und danach 0.31 g Phophorpentaxchlorid (1.5 mmol) eingetragen. Nach 15 Minuten bei dieser Temperatur wurde im Vakuum das Lösungsmittel abgezogen und mit Methylenchlorid/Aceton (1 + 1) nachdestilliert. Danach nahm man den Rückstand in 14 ml Aceton auf und tropfte die Lösung bei 0°C zu einer Lösung aus 0.58 g (1.5 mmol) 7-Amino-3-[(4-methoxycarbonyl-1,3-thiazol-5-yl)thiomethyl]ceph-3-em-4-carbonsäure (Herstellungsbeispiel 10 in 14 ml Aceton und 20 ml Wasser, der zuvor 165 mg Natriumhydrogencarbonat und 0.56 ml Triethylamin zugesetzt wurden. Nach 30 Minuten bei 0°C und 1 h Rühren bei Raumtemperatur wurde mit 2N Salzsäure auf pH 3 gestellt und dreimal mit je 100 ml Essigester extrahiert. Die organische Phase wurde getrocknet ($MgSO_4$) und i.Vak. eingeengt. Nach Chromatographie ($SiO_2$; Chloroform, Cyclohexan, Ethanol, Eisessig = 5 + 3 + 1 + 0.5) wurden 250 mg der gewünschten Titelverbindung erhalten, das in der nächsten Stufe weiterverarbeitet wurde.

**Stufe 2**

7-[2-(2-Aminothiazol-4-yl)-2-(Z)-hydroxyimino-acetamido]-3-[(4-methoxycarbonyl-1,3-thiazol-5-yl)thiomethyl]-

ceph-3-em-4-carbonsäure

250 mg der in Stufe 1 erhaltenen Verbindung wurden in 20 ml 80 %-iger Ameisensäure gelöst und 90 Minuten bei Raumtemperatur gerührt. Danach wurde vom entstandenen Triphenylcarbinol abgesaugt und i. Vak. eingeengt. Der Rückstand wurde mehrmals in Toluol aufgenommen und das Lösungsmittel i. Vak. abgezogen. Danach löste man den Rückstand mit 20 ml verdünnter Natriumhydrogencarbonatlösung auf und säuerte mit 2N Salzsäure auf pH 3 an, saugte ab und trocknete i. Vak. (Phosphorpentoxid). Man erhält 80 mg der gewünschten Titelverbindung.

Fp.: 112°C (unter Zersetzung)

IR (KBr): 1770, 1535, 1260, 1045 $cm^{-1}$.

$^1$H-NMR (270 MHz, DMSO-$d_6$): δ(ppm) = 3.5 und 3.73 (2H,AB,J = 18Hz,$SCH_2$), 3.65 (3H,s,$OCH_3$), 4.0 und 4.28 (2H,AB,J = 12Hz, 3′-$CH_2$), 5.13 (1H,d,J = 6Hz,6-H), 5.76 (1H,dd,J = 6Hz,7-H), 6.68 (1H,s,Aminothiazol H), 7.15 (2H,bs,$NH_2$), 7.6 (2H,s,Amid $NH_2$), 9.45 (1H,d,J = 18Hz,NHCO), 11.3 (1H,bs,Oxim-H).

**Ausführungsbeispiel 5**

7-[2-(2-Aminothiazol-4-yl)-2-(Z)-hydroxyimino-acetamido]-3-[(4-carbamoyl-1,3-thiazol-5-yl)thionethyl]ceph-3-em-4-carbonsäure

**Stufe 1**

7-[2-(2-Tritylaminothiazol-4-yl)-2-(Z)-trityl-oximino-acetamido]-3-[4-carbamoyl-1,3-thiazol-5-yl) thiomethyl]-ceph-3-em-4-carbonsäure

Zu einer Lösung von 1,88 g (2.8 mmol) 2-(2-Tritylaminothiazol-4-yl)-2-(Z)-trityl-oximino-essigsäure in 20 ml wasserfreiem Methylenchlorid wurden 0.37 ml (1 eq.) Triethylamin zugetropft, auf -20°C gekühlt und anschließend 0.56 g (2.8 mmol) Phosphorpentachlorid eingetragen. Nach 15 Minuten bei dieser Temperatur wurde im Vakuum das Lösungsmittel abgezogen und mit Methylenchlorid/Aceton (1 + 1) nachdestilliert. Anschließend nahm man den Rückstand in 14 ml Aceton auf und tropfte die Lösung bei 0°C zu einer Lösung von 0.75 g (2 mmol) 7-Amino-3-[(4-carbamoyl-1,2-thiazol-5-yl)thiomethyl] ceph-3-em-4-carbonsäure (Herstellungsbeispiel 11) in 14 ml Aceton und 20 ml Wasser, der zuvor 220 mg Natriumhydrogencarbonat und 0.74 ml Triethylamin zugesetzt wurden. Nach 30 Minuten bei 0°C und 1 h Nachrühren bei Raumtemperatur wurde mit 2N Salzsäure auf pH 3 gestellt und dreimal mit je 75 ml Essigester extrahiert. Die organische Phase wurde getrocknet ($MgSO_4$) und i.Vak. eingeengt. Nach Chromatographie ($SiO_2$; Chloroform, Cyclohexan, Ethanol, Eisessig = 5 + 3 + 1 + 0.5) wurden 0.9 g der gewünschten Titelverbindungen erhalten, die in der nächsten Stufe weiterverarbeitet wurde.

**Stufe 2**

7 [2-(2-Aminothiazol-4-yl)-2-(Z)-hydroxyimino-acetamido]-3-[(4-carbamoyl-1,3-thiazol-5-yl)thiomethyl]ceph-3-em-4-carbonsäure

900 mg (0.87 mmol) der in Stufe 1 erhaltenen Verbindung wurden in 25 ml 80 %-iger Ameisensäure gelöst und 90 Minuten bei Raumtemperatur gerührt. Anschließend wurde von entstandenem Triphenylcarbinol abgesaugt und i.Vak. eingeengt. Danach löste man den Rückstand mit 20 ml verdünnter Natriumhydrogencarbonatlösung, filtrierte und säuerte danach mit 2N Salzsäure auf pH 3 an. Der Niederschlag wurde sodann abgesaugt und i. Vak. getrocknet (Phosphorpentoxid). Man erhielt 280 mg (59 %) der gewünschten Titelverbindung.

Fp.: 118°C (unter Zersetzung)

IR: 1780,1540,1265,1045 $cm^{-1}$

$^1$H-NMR (270 MHz,DMSO-$d_6$): δ(ppm) = 3.5 und 3.72 (2H,AB,J = 18Hz,$SCH_2$), 4.0 und 4.28

(2H,AB,J = 12Hz,3′CH2) 5.13 (1H,d,J = 6Hz,6-H), 5.76 (1H,dd,J = 6Hz,7-H), 6.66 (1H,s,Aminothiazol-H), 7.15 (2H,bs,$NH_2$), 7.6 (2H,s,Amid-$NH_2$), 9.45 (1H,d,J = 8Hz,NHCO), 11.3 (1H,bs,Oxim-H).

**Ausführungsbeispiel** 6

7-[2-(2-Aminothiazol-4-yl)-2-(Z)-carboxymethoxyimino-acetamido -3-[(4-pyrrolidinocarbonyl-1,3-thiazol-5-yl)-thiomethyl]ceph-3-em-4-carbonsäure-trifluoracetat

**Stufe 1**

7-[2-(2-Aminothiazol-4-yl)-2-(Z)-carboxy-tert-butyl-methoxyiminoacetamido]-3-[(4-pyrrolidinocarbonyl-1,3-thiazol-5-yl)thiomethyl]ceph-3-em-4-carbonsäure

Zu einer Lösung von 600 mg (2 mmol) 2-Aminothiazol-4-yl-2-(Z)-carboxy-tert-butyl-methoxyiminoessigsäure in 30 ml Dimethylformamid wurden 300 mg (2 mmol) N-Hydroxy-benzotriazol und 450 mg (2.2 mmol) Dicyclohexylcarbodiimid gegeben und 30 Minuten nachgerührt. Danach wurden 825 mg (2 mmol) 7-Amino-3-[(4-pyrrolidinocarbonyl-1,3-thiazol-5-yl)thiomethyl]ceph-3-em-4-carbonsäure (Herstellungsbeispiel 13) zugegeben und 8 h bei Raumtemperatur gerührt. Danach saugte man von entstandenem Dicyclohexylharnstoff ab und entfernte das Lösungsmittel i. Vak. (0.1 Torr). Der Rückstand wurde sodann mit 0.1N Natriumhydrogencarbonatlösung gelöst und die Lösung mit Essigester extrahiert. Die Phasen wurden anschließend getrennt und die wässrige Lösung mit 2N Salzsäure auf pH 3 gestellt. Das Produkt wurde abgesaugt und ohne weitere Reinigung in der nächsten Stufe weiterverarbeitet.

Fp.: 180 °C (unter Zersetzung)

**Stufe 2**

7-[2-(2-Aminothiazol-4-yl)-2-(Z)-carboxymethoxyimino-acetamido]-3-[(4-pyrrolidinocarbonyl-1,3-thiazol-5-yl) thiomethyl]ceph-3-em-4-carbonsäure-trifluoracetat

400 mg (0.56 mmol) der Stufe 1 wurden in 10 ml Trifluoressigsäure gelöst und 30 Minuten nachgerührt. Danach wurde das Lösungsmittel abgezogen und der Rückstand mit Diethylether ausgerührt. Man erhielt so 363 mg der gewünschten Titelverbindung.

Fp.: 170°C (unter Zersetzung)

IR (KBr): 1780, 1670, 1630, 1190, 1045 $cm^{-1}$.

$^1$H-NMR (270 MHz, DMSO-$d_6$): δ(ppm) = 1.83 (8H,bs,Pyrrolidinocarbonyl), 3.5 und 3.75 (2H,AB,J = 18Hz,$SCH_2$), 3.95 und 4.2 (2H,AB,J = 12Hz,3′-$CH_2$), 4.62 (2H,s,$CH_2CO_2H$), 5.16 (1H,d,J = 6.5Hz,6-H), 5.77 (1H,dd,J = 6.5xH2,7-H), 6.75 (1H,s,Aminothiazol H), 9.12 (1H,s,Thiazol-H von 3′-Het).

Ausführungsbeispiel 7

7-[2-(2-Aminothiazol-4-yl)-2-(Z)-methoxyimino-acetamido]-3-[4-methoxycarbonyl-1,3-thiazol-5-yl)thiomethyl]-ceph-3-am-4-carbonsäure-2,2-dimethyl-propionyloxymethylester

Zu einer Lösung von 1,22 g (2mmol) 7-[2-(2-Aminothiazol-4-yl)-2-(Z)-methoxyimino-acetamido]-3-[(4-methoxycarbonyl-1,3-thiazol-5-yl)thiomethyl] ceph-3-em-4-carbonsäure-Kaliumsalz in 15 ml wasserfreiem DMF wurden bei 0°C 0,6g (2,8mmol) 2,2-Dimethylpropansäure-jodmethyl-ester getropft und 2,5 h bei dieser Temperatur nachgerührt. Anschließend wurde auf 100 ml 2 %-ige Natriumhydrogencarbonat-lösung gegossen und dreimal mit je 100 ml Essigester extrahiert. Die vereinigten organischen Lösungen wurden sodann mit Wasser und gesättigter Natriumchloridlösung gewaschen, über $Na_2SO_4$ getrocknet und eingeengt. Man erhielt 0,7g eines obigen Produktes, das mit Diethylether verrieben wurde. Das feste Produkt wurde

abgesaugt und über Phosphorpentoxid i. Vak. getrocknet. Man erhielt 300mg der gewünschten Titelverbindung

Fp.: 120°C (unter Zersetzung)

IR (KBr): 1780, 1750, 1675, 1110 $cm^{-1}$.

$^1$H-NMR (270 MHz, DMSO-$d_6$): δ(ppm) = 1.15 (9H, s, tert.-Butyl), 3.75 (2H, AB, J = 18Hz,$SCH_2$),3.85 und 3.87 (6H, 2x s, $CO_2CH_3$ und =N-$OCH_3$), 4.2 (2H, AB, J = 12 Hz, 3'-$CH_2$), 5.2 (1H, d, J = 5Hz, 6-H), 5.78 und 5.9 (3H, AB und q, J = 5Hz, -$CH_2$OCO und 7-H), 6.75 (1H, s, Thiazol-H), 7.25 (2H, bs, $NH_2$), 9.05 (1H, s, -S-CH = N), 9.65 (1H, d, J = 8Hz, NHCO).

**Ausführungsbeispiel 8**

7-[2-(2-Aminothiazol-4-yl)-2-(Z)-methoxyimino-acetamido]-3-[(4-carboxy-1,3-thiazol-5-yl)thiomethyl]ceph-3-em-4carbonsäure

Die Herstellung erfolgte analog zu Ausführungsbeispiel 1.

IR (KBr): 3500-2500, 1790, 1700, 1046 $cm^{-1}$.

$^1$H-NMR (270 MHz, DMSO-$d_6$): δ (ppm) = 3.56 und 3.75 (2H, AB, J = 18Hz, $SCH_2$), 3.83 (2H, S, =N-$OCH_3$), 4.15 und 4.31 (2H, AB, J = 12Hz, 3'-$CH_2$), 5.15 (1H, d, J = 6 Hz, 6-H), 5.78 (1H, dd, 3 = 6 Hz, 7-H), 6.71 (1H, s, Thiazol-H), 7.2 (2H, s, $NH_2$), 9.05 (1H, s, -S-CH = N), 9.60 (1H, d, NH), 11.1 (1H, 2, $CO_2$H).

**Ansprüche**

1. Cephalosporinderivate der allgemeinen Formel (I)

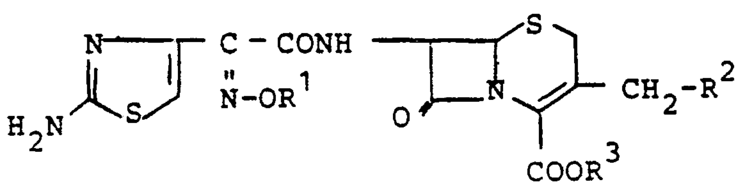

(I)

und deren physiologisch verträgliche Salze und Säureadditionssalze, worin

$R^1$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_4$-$C_7$-Cycloalkenyl, die Gruppe

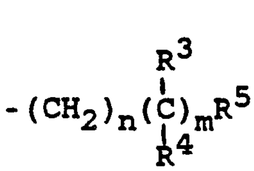

,

worin m oder n jeweils 0 oder 1, $R^3$ und $R^4$ gleich oder verschieden sein können und Wasserstoff, Aryl, eine $C_1$-$C_4$-Alkylgruppe, oder zusammen mit dem Kohlenstoffatom, an das sie gebunden sind, eine Methylen- oder eine $C_3$-$C_7$-Cycloalkylidengruppe bilden; $R^5$ eine Gruppe -$CO_2R^6$, in der $R^6$ Wasserstoff, $C_1$-$C_4$-Alkyl oder ein Äquivalent eines Alkalimetalls, Erdalkalimetalls, Ammonium oder einer organischen Aminbase bedeutet;

$R^2$ für einen 5-Thio-1,3-thiazolrest der Formel (II)

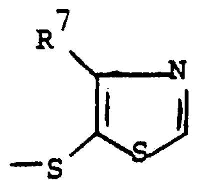

(II)

steht, worin $R^7$ die Bedeutung besitzt von Wasserstoff, Carboxy, $C_1$-$C_4$-Alkoxycarbonyl, Carbamoyl, in dem die Aminogruppe gegebenenfalls auch einfach oder zweifach durch $C_1$-$C_4$-Alkyl substituiert sein kann oder auch zwei der am Stickstoff stehenden Alkylgruppen zu einem Di- bis Pentamethylenring geschlossen sein können und

$R^3$ Wasserstoff, ein physiologisch verträgliches Kation oder eine leicht abspaltbare Estergruppe bedeutet und in denen die $R^1$O-Gruppe in syn-Position steht.

2. 7-[2-(2-Aminothiazol-4-yl)-2-(Z)-methoxyimino-acetamido]-3-[4-methoxycarbonyl-1,3-thiazol-5-yl)-thiomethyl]ceph-3-em-4-carbonsäure.

3. 7-[2-(2-Aminothiazol-4-yl)-2-(Z)-methoxyimino-acetamido]-3-[(4-carbamoyl-1,3-thiazol-5-yl)thiomethyl]-ceph-3-em-4-carbonsäure.

4. 7-[2-(2-Aminothiazol-4-yl)-2-(Z)-methoxyimino-acetamido]-3-[(1,3-thiazol-5-yl)thiomethyl]ceph-3-em-4-carbonsäure.

5. 7-[2-(2-Aminothiazol-4-yl)-2-(Z)-hydroxyimino-acetamido]-3-[(4-methoxycarbonyl-1,3-thiazol-5-yl)-thiomethyl]ceph-3-em4carbonsäure.

6. 7-[2-(2-Aminothiazol-4-yl)-2-(Z)-hydroxyimino-acetamido]-3-[(4-carbamoyl-1,3-thiazol-5-yl)thiomethyl]-ceph-3-em-4-carbonsäure.

7. Verfahren zur Herstellung von Verbindungen der allgemeinen Formel (I) und ihrer physiologisch verträglichen Salze und Säureadditionssalze, dadurch gekennzeichnet, daß man

a) eine Verbindung der allgemeinen Formel (III)

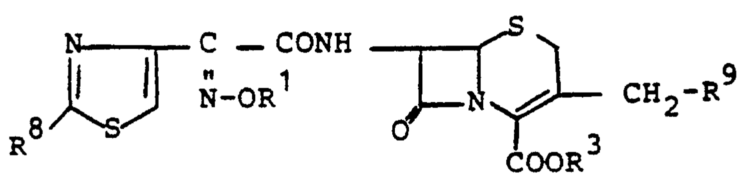

(III)

oder deren Salze, worin $R^1$ und $R^3$ die obengenannte Bedeutung hat, $R^8$ eine Amino- oder eine geschützte Aminogruppe bedeutet und $R^9$ eine durch 5-Thio-1,3-thiazol oder in Position 4 substituiertes 5-Thio-1,3-thiazol, die den Resten $R^2$ in der Formel (I) entsprechen, austauschbare Gruppe bedeutet, mit einem solchen 5-Mercapto-1,3-thiazol oder einem seiner 4-substituierten Derivate umsetzt, eine gegebenenfalls vorhandene Schutzgruppe abspaltet und falls erforderlich, das erhaltene Produkt in ein physiologisch verträgliches Salz oder Säureadditionssalz überführt, oder

b) eine 7-Amino-cephemverbindung der allgemeinen Formel (IV)

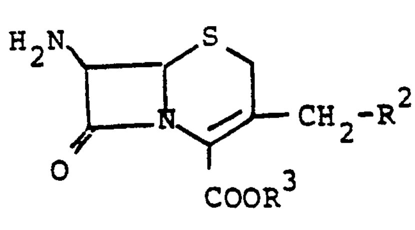

(IV)

oder deren Salze und Säureadditionssalze, worin $R^2$ und $R^3$ die obengenannte Bedeutung haben und worin die Aminogruppe auch in Form eines reaktionsfähigen Derivats vorliegen kann, mit einer Thiazol-4-yl-2-syn-oximessigsäure der allgemeinen Formel (V),

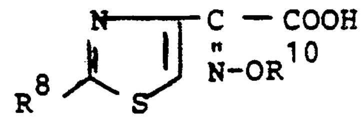

(V)

worin $R^8$ die obige Bedeutung besitzt und $R^{10}$ die Bedeutung von $R^1$ hat, wobei für den Fall, daß $R^1$ in Formel (I) Wasserstoff ist, $R^{10}$ eine Schutzgruppe bedeutet oder mit einem aktivierten Derivat dieser Verbindung umsetzt, eine gegebenenfalls vorhandene Schutzgruppe abspaltet und falls erforderlich, das erhaltene Produkt in ein physiologisch verträgliches Salz oder Säureadditionssalz überführt.

8. Verbindungen der allgemeinen Formel (VI)

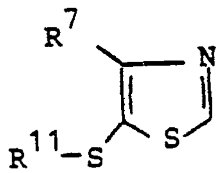

(VI)

worin $R^7$ die in Anspruch 1 genannte Bedeutung besitzt und $R^{11}$ für Wasserstoff, Natrium oder Kalium steht.

9. Arzneimittel, gekennzeichnet durch einen Gehalt an einer Verbindung gemäß Anspruch 1.

10. Verwendung einer Verbindung gemäß Anspruch 1, zur Herstellung eines Arzneimittels mit antibakterieller Wirkung.

Patentansprüche für folgende Vertragsstaaten: ES, GR

1. Verfahren zur Herstellung von Verbindungen der allgemeinen Formel (I)

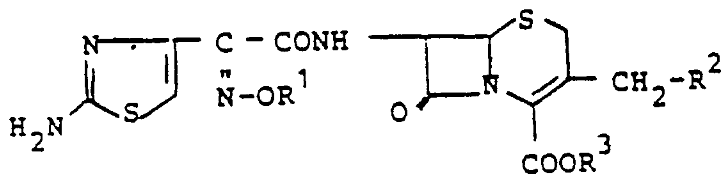

(I)

und ihrer physiologisch verträglichen Salze und Säureadditionssalze, worin

$R^1$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_4$-$C_7$-Cycloalkenyl, die Gruppe

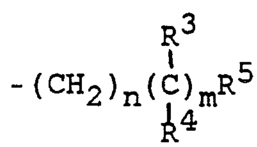

,

worin m oder n jeweils 0 oder 1, $R^3$ und $R^4$ gleich oder verschieden sein können und Wasserstoff, Aryl, eine $C_1$-$C_4$-Alkylgruppe, oder zusammen mit dem Kohlenstoffatom, an das sie gebunden sind, eine Methylen- oder eine $C_3$-$C_7$-Cycloalkylidengruppe bilden; $R^5$ eine Gruppe -$CO_2R^6$, in der $R^6$ Wasserstoff, $C_1$-$C_4$-Alkyl oder ein Äquivalent eines Alkalimetalls, Erdalkalimetalls, Ammonium oder einer organischen Aminbase bedeutet;

$R^2$ für einen 5-Thio-1,3-thiazolrest der Formel (II)

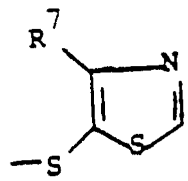

(II)

steht, worin $R^7$ die Bedeutung besitzt von Wasserstoff, Carboxy, $C_1$-$C_4$- Alkoxycarbonyl, Carbamoyl, in dem Aminogruppe gegebenfalls auch einfach oder zweifach durch $C_1$-$C_4$-Alkyl substituiert sein kann oder auch zwei der am Stickstoff stehenden Alkylgruppen zu einem Di- bis Pentamethylenring geschlossen sein können und

$R^3$ Wasserstoff, ein physiologisch verträgliches Kation oder eine leicht abspaltbare Estergruppe bedeutet und in denen die $R^1$O-Gruppe in syn-Position steht,

dadurch gekennzeichnet, daß man

a) eine Verbindung der allgemeinen Formel (III)

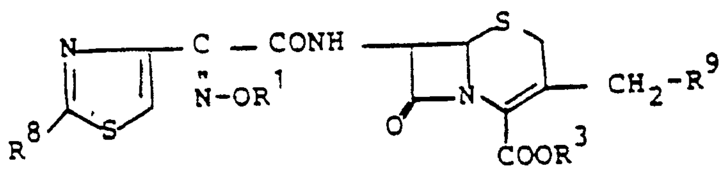

(III)

oder deren Salze, worin $R^1$ und $R^3$ die obengenannte Bedeutung hat, $R^8$ eine Amino- oder eine geschützte Aminogruppe bedeutet und $R^9$ eine durch 5-Thio-1,3-thiazol oder in Position 4 substituiertes 5-Thio-1,3-thiazol, die den Resten $R^2$ in der Formel (I) entsprechen, austauschbare Gruppe bedeutet, mit einem solchen 5-Mercapto-1,3-thiazol oder einem seiner 4-substituierten Derivate umsetzt, eine gegebenenfalls vorhandene Schutzgruppe abspaltet und falls erforderlich, das erhaltene Produkt in ein physiologisch verträgliches Salz oder Säureadditionssalz überführt, oder

b) eine 7-Amino-cephemverbindung der allgemeinen Formel (IV)

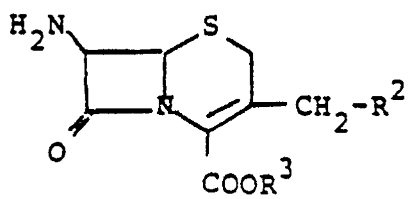

(IV)

oder deren Salze und Säureadditionssalze, worin $R^2$ und $R^3$ die obengenannte Bedeutung haben und worin die Aminogruppe auch in Form eines reaktionsfähigen Derivats vorliegen kann, mit einer Thiazol-4-yl-2-syn-oximessigsäure der allgemeinen Formel (V),

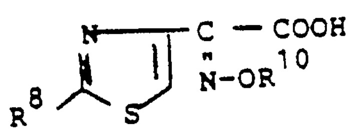

(V)

worin $R^8$ die obige Bedeutung besitzt und $R^{10}$ die Bedeutung von $R^1$ hat, wobei für den Fall, daß $R^1$ in Formel (I) Wasserstoff ist, $R^{10}$ eine Schutzgruppe bedeutet oder mit einem aktivierten Derivat dieser Verbindung umsetzt, eine gegebenenfalls vorhandene Schutzgruppe abspaltet und falls erforderlich, das erhaltene Produkt in ein physiologisch verträgliches Salz oder Säureadditionssalz überführt.

2. Verwendung einer Verbindung gemäß Anspruch 1, zur Herstellung eines Arzneimittels mit antibakterieller Wirkung.

Europäisches Patentamt

# EUROPÄISCHER RECHERCHENBERICHT

Nummer der Anmeldung

**EP 90 11 3195**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 289 002 (MEIJI SEIKA KAISHA LTD.) * Ansprüche 1-4,25,30; Seite 27, Beispiele 32,33; Seite 29, Beispiel 41 * | 1,7-10 | C 07 D 501/36<br>C 07 D 277/36<br>C 07 D 277/56<br>A 61 K 31/545 |
| A | EP-A-0 148 007 (YAMANOUCHI PHARMACEUTICAL CO. LTD.) * Ansprüche 1,5,7; Zusammenfassung; Beispiel 8 * | 1,7,9,10 | |
| A | EP-A-0 176 369 (YAMANOUCHI PHARMACEUTICAL LTD.) * Ansprüche 1,2,4,8; page 4, lines 1-24 * | 1,7,9,10 | |
| A | EP-A-0 186 463 (E. LILLY AND CO.) * Ansprüche 1,7,8 * | 1,9,10 | |
| A,D | DE-A-2 804 040 (HOECHST AG) * Anspruch 1 * | 7 | |

RECHERCHIERTE SACHGEBIETE (Int. Cl.5)

C 07 D 277/00
C 07 D 501/00

Der vorliegende Recherchenbericht wurde für alle Patentansprüche erstellt

| Recherchenort | Abschlußdatum der Recherche | Prüfer |
|---|---|---|
| Berlin | 24 Oktober 90 | HASS C V F |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument